# FASCICULE DE BREVET EUROPEEN

(11) **EP 1 175 899 B1**
(45) Date de publication et mention de la délivrance du brevet: **29.08.2007**
(21) Numéro de dépôt: 00402159.8
(22) Date de dépôt: 27.07.2000
(51) Int. Cl.: A61K 9/16

(54) **Granules à base d'amidon et de lactose**
Granulat bestehend aus Stärke und Laktose
Granules consisting of starch and lactose

(43) Date de publication de la demande: 30.01.2002
(73) Titulaire: Roquette Frères, 62136 Lestrem (FR)
(72) Inventeur: Luhn, Oliver, 83512 Wasserburg (DE)
(74) Mandataire: Boulinguiez, Didier

(56) Documents cités:
- DE-A- 4 400 295
- GB-A- 833 458
- GB-A- 2 093 052
- US-A- 5 006 345
- HILL PM: "Starch paste granulations: binder dilution effects on granulations and tablets" JOURNAL OF PHARMACEUTICAL SCIENCES, vol. 65, no. 2, 1976, pages 313-314, XP002156442
- TADASHI MAKINO ET AL: "IMPORTANCE OF GELATINIZATION DEGREE OF STARCH PASTE BINDER IN HARDNESS AND DISINTEGRATION TIME OF TABLETS" CHEMICAL AND PHARMACEUTICAL BULLETIN,JP,PHARMACEUTICAL SOCIETY OF JAPAN. TOKYO, vol. 43, no. 3, 1 mars 1995 (1995-03-01), pages 514-516, XP000498294 ISSN: 0009-2363
- VISAVARUNGROJ N ET AL: "CROSSLINKED STARCH AS BINDING AGENT I. CONVENTIONAL WET GRANULATION" INTERNATIONAL JOURNAL OF PHARMACEUTICS (AMSTERDAM), vol. 59, no. 1, 1990, pages 73-78, XP000971702 ISSN: 0378-5173

## Description

La présente invention a pour objet une composition de granules à base d'amidon et de lactose conformément aux revendications 1 à 3 ainsi que le procédé permettant d'obtenir ces granules conformément à la revendication 4.

Elle a également pour objet l'utilisation de tels granules notamment comme excipient dans l'industrie pharmaceutique ou alimentaire ou comme additif ou support dans les industries agro-alimentaires ou chimiques.

L'industrie pharmaceutique est consommatrice de tonnages importants d'amidon et de lactose. Ceux-ci sont notamment utilisés en tant qu'excipient dans les formes sèches que sont par exemple les poudre de remplissage des gélules, les poudres à dissoudre, les préparations nutritives pulvérulentes et les comprimés.

L'industrie alimentaire utilise quant à elle ce type d'excipient notamment dans les aliments ou boissons à disperser et à diluer.

En ce qui concerne le domaine auquel on s'intéressera spécifiquement dans la présente invention, à savoir les excipients pharmaceutiques ou alimentaires, de tels excipients existent sous forme de poudres obtenues par granulation humide d'un mélange de lactose et d'amidon, additionné de liants ou de surfactants. Ces poudres possèdent généralement des propriétés peu satisfaisantes pour la compression directe.

Les caractères les plus importants pour apprécier l'aptitude pour la compression directe d'une poudre sont l'aptitude à l'écoulement (alimentation régulière de la chambre de compression à partir de la trémie), la résistance à l'abrasion (ou non-friabilité) et la cohésion après compactage des particules qui conditionne la dureté des comprimés. Les comprimés produits doivent être suffisamment durs pour résister à la casse mais en même temps avoir de bonnes propriétés de désintégration lorsqu'ils se trouvent dans le milieu gastrique.

Parmi les excipients les plus couramment rencontrés en compression, on peut citer notamment la cellulose, l'amidon, le lactose, le phosphate dicalcique.

La cellulose microcristalline remplit toutes les conditions attendues d'un excipient de compression directe, mais elle reste difficile à produire et relativement onéreuse. Elle présente en outre l'inconvénient de provoquer une diminution de la dureté des comprimés consécutivement à une reprise en eau au cours du stockage. De plus, elle procure une sensation désagréable en bouche.

Le lactose est un diluant très employé dans la technologie des comprimés. Il se présente sous deux formes principales : cristallisé ou atomisé. Le lactose cristallisé se présente sous trois formes cristallines différentes : l'α-lactose anhydre (non disponible dans le commerce), le β-lactose anhydre (généralement appelé lactose anhydre), et l'α-lactose monohydrate. Le lactose anhydre présente l'inconvénient d'être hygroscopique ce qui entraîne des problèmes de stabilité dans le temps. L'α-lactose monohydrate est stable, mais sa comprimabilité et son temps de désintégration sont limités. Afin d'améliorer ses propriétés en compression, le lactose a été modifié par atomisation et agglomération.

Le lactose atomisé est hautement compressible, et la sphéricité de ses particules lui confère des propriétés d'écoulement satisfaisantes. Il est moins stable et sa durée de conservation est plus courte que celle du lactose cristallisé. Il ne possède en outre pas de propriétés désintégrantes. Les comprimés fabriqués à partir de lactose atomisé développent au stockage une coloration jaunâtre plus intense que celle développée par le lactose monohydraté.

Le lactose aggloméré est une poudre stable qui s'écoule bien, mais moins compressible que le lactose atomisé.

La comprimabilité du lactose reste insuffisante et a pu être améliorée en lui ajoutant un excipient liant ou diluant possédant une comprimabilité meilleure comme une cellulose microcristalline. Ces mélanges se compriment facilement moyennant l'addition d'un lubrifiant. Les celluloses microcristallines ont cependant l'inconvénient d'un prix élevé, et d'une baisse de la dureté des comprimés formés consécutivement à une prise d'humidité.

Quant à l'amidon, il présente du fait de la faible taille de ses particules et de sa faible densité, l'inconvénient de ne pas s'écouler. L'élasticité importante de ses granules lui confère une piètre comprimabilité qui ne permet pas la fabrication de comprimés de dureté satisfaisante. Par contre, il possède de bonnes propriétés désintégrantes du fait de son pouvoir gonflant dans l'eau. L'amidon peut également servir de liant et de diluant et même d'agent favorisant l'écoulement.

On a proposé d'associer le lactose et l'amidon par granulation humide (BODE-TUNJI M.O., JAIYEOBA K.T., Labo-Pharma - Probl. Tech. - 32, n°340, Mars 1984, pp 190-192). Cependant, il a été démontré que l'addition d'amidon au lactose réduit la taille moyenne des granulés et augmente leur friabilité, cet effet étant compensé par l'addition d'un surfactant.

On a également proposé de granuler du lactose et de l'amidon en utilisant un polymère synthétique comme agent liant (WAN L.C., LIM K.S., S.T.P. Pharma Sciences 1 (5) 285-293, 1991). Les compositions obtenues présentent un écoulement acceptable, mais des densités relativement faibles.

On connaît en outre des procédés de préparation par atomisation d'excipients de compression complexes, tels que celui décrit dans le brevet US 5.006.345, comprenant du lactose, un liant tel que la polyvinylpyrrolidone ou l'hydroxypropylméthylcellulose, et un désintégrant tel que notamment la carboxyméthylcellulose réticulée ou l'amidon carboxyméthylé.

Il reste donc un besoin non satisfait pour une composition de granules d'amidon et de lactose, possédant une friabilité réduite, un écoulement performant, une bonne comprimabilité et des propriétés désintégrantes satisfaisantes, tout en étant peu hygroscopique.

L'invention a pour but de remédier à ce besoin et la Demanderesse a eu le mérite de trouver, après de nombreux travaux que ce but pouvait être atteint dès lors qu'on utilise des granules conformes à l'invention, à base d'amidon et de lactose. Pour obtenir de tels granules, la Demanderesse a constaté que, contre toute attente, il convenait d'employer un mélange de lactose et d'amidon granulaire et de modifier ses caractéristiques physiques en employant un procédé approprié de telle sorte que l'on obtienne simultanément une friabilité modérée, une comprimabilité satisfaisante, un écoulement performant, tout en conservant des propriétés désintégrantes.

L'invention a donc pour objet des granules consistant en lactose et en amidon caractérisés en ce que le ratio lactose/amidon est compris entre 90/10 et 25/75, et en ce qu'ils présentent une friabilité inférieure ou égale à 80% selon un test A.

Ce test A consiste à soumettre les granules à tester à une action mécanique dans un appareil dénommé friabilimètre. On utilise pour cela un appareil de marque ERWEKA TA fabriqué par la société ERWEKA (RFA-63150 HEUSENSTAMM) tournant à une vitesse de rotation uniforme de 25 tours/minute, et équipé d'un tambour d'abrasion dans lequel on a introduit 5 billes d'acier identiques d'un diamètre de 17 mm et d'un poids de 18,87g. Pour réaliser ce test A, on introduit dans le tambour d'abrasion de ce friabilimètre, une quantité de 15 g de produit de granulométrie comprise entre 100 et 200 µm, puis on met l'appareil en rotation pendant 5 minutes.

A la fin de l'expérience, on détermine la proportion pondérale représentée par le résidu retenu sur un tamis d'une largeur de maille de 100 microns.

La valeur de la friabilité correspond au pourcentage de poudre non retenu par le tamis précédemment défini. La friabilité est d'autant plus grande que le pourcentage de poudre non retenue par le tamis est grand.

Les granules conformes à l'invention présentent selon ce test A une friabilité modérée, c'est à dire inférieure ou égale à 80% et de préférence inférieure ou égale à 60%.

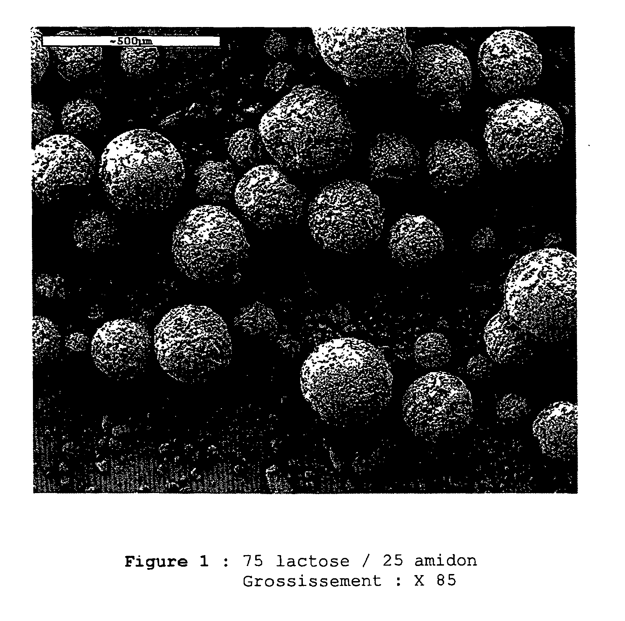

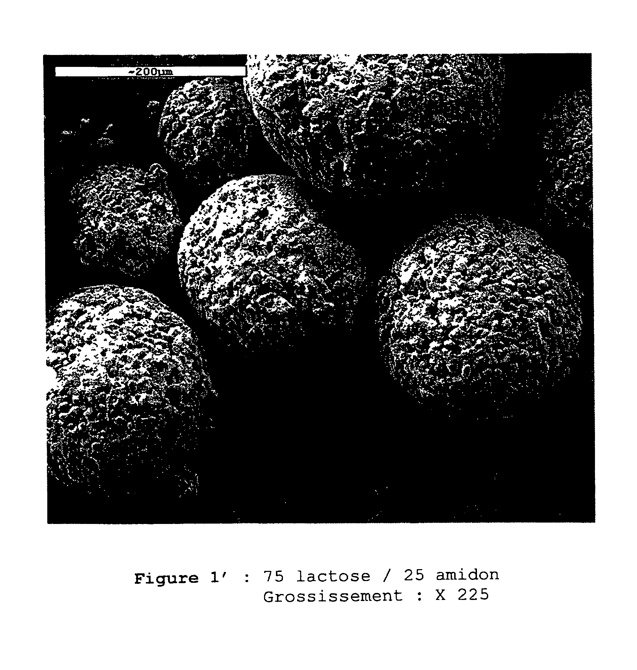

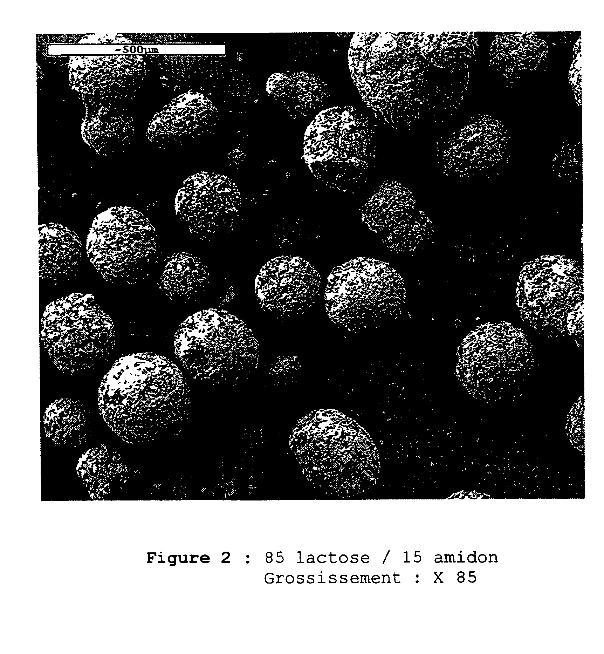

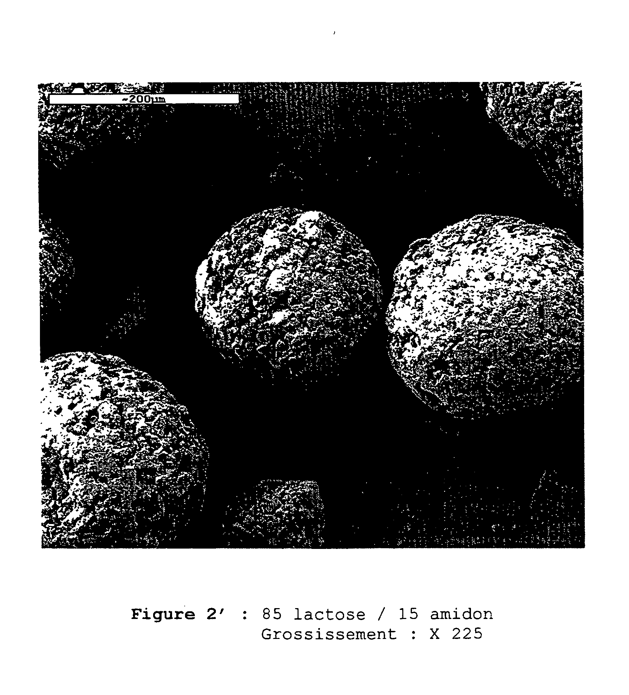

Les granules conformes à l'invention sont également caractérisés par une structure sphérique lorsqu'ils sont observés en microscopie électronique à balayage. Cette structure tout à fait particulière est illustrée par les figures 1, 1' et 2, 2' qui mettent en évidence la sphéricité parfaite des granules obtenus. La figure 1 représente une photographie au microscope électronique à balayage d'un granule conforme à l'invention dont le ratio de lactose et d'amidon est de 75/25. La figure 2 représente une photographie au microscope électronique à balayage d'un granule conforme à l'invention dont le ratio de lactose et d'amidon est de 85/15. Grâce à cette structure sphérique, les granules conformes à l'invention présentent un écoulement tout à fait satisfaisant.

Par lactose on entend le lactose monohydrate tel que défini par la pharmacopée européenne, 1997,0187-p.1111-1112. Par amidon, on entend les amidons de toutes origines, naturels ou hybrides, de type granulaire. On utilisera de préférence un amidon de maïs natif, et en particulier un amidon de maïs blanc tel que celui commercialisé par la Demanderesse sous l'appellation « amidon extra-blanc » qui permet d'obtenir des granules d'une blancheur tout à fait satisfaisante.

Au-delà de 90% de lactose par rapport à l'amidon contenu dans les granules, la Demanderesse a constaté que lesdits granules ne possédaient pas de propriétés désintégrantes satisfaisantes. En deçà de 25% de lactose, soit pour plus de 75% d'amidon dans les granules, ceux-ci ne se compriment plus correctement en raison des propriétés élastiques de l'amidon et les propriétés d'écoulement ne sont pas satisfaisantes.

De préférence, on choisira un ratio de lactose et d'amidon compris entre 85/15 et 50/50.

Les granules conformes à l'invention peuvent être caractérisés par leur comprimabilité déterminée selon un test B.

Ce test B consiste à mesurer les forces, exprimées en Newton, qui sont représentatives de la comprimabilité de la composition de granules étudiée, données pour deux valeurs différentes de densité de comprimés. Ces forces traduisent la résistance à l'écrasement de comprimés qui sont cylindriques à faces convexes (rayon de courbure de 14mm), d'un diamètre de 13 mm, d'une épaisseur de 6 mm et de masses volumiques apparentes de 1,3 et 1,4 g/ml. Cette comprimabilité exprimée à de telles densités de comprimés reflète bien les propriétés avantageuses des granules selon l'invention en matière de compression directe.

Pour effectuer ce test B, on prépare des comprimés à partir de granules conformes à l'invention auxquels on ajoute au préalable 0,5% en poids de stéarate de magnésium en tant que lubrifiant.

La compression est effectuée grâce à une presse alternative FROGERAIS de type AM. Cette presse est équipée de poinçons ronds à faces concaves, d'un diamètre égal à 13mm.

On règle sur la presse l'enfoncement du poinçon supérieur et le volume de remplissage de la matrice, de façon à faire varier la densité des comprimés, et on détermine la dureté correspondante de ces comprimés en utilisant un duromètre ERWEKA de type TBH 30 GMD.

Les granules conformes à l'invention dont le ratio de lactose et d'amidon est compris entre 90/10 et 50/50 présentent une comprimabilité remarquable, supérieure à celle des produits de l'art antérieur, se traduisant par une dureté de comprimés supérieure à 70 N et de préférence supérieure à 80 N pour une densité de 1,3 g/ml, et/ou supérieure à 170 N et de préférence supérieure à 180 N pour une densité de 1,4 g/ml.

Les granules selon la présente invention se caractérisent en outre par un angle de repos inférieur à 45°, et de préférence inférieur à 40°. La détermination de l'angle de repos d'une poudre est une des méthodes les plus généralement employées pour apprécier ses propriétés d'écoulement. Il s'agit de mesurer la hauteur d'un cône formé par la poudre qui s'est écoulée d'un cylindre de volume connu, et d'en déduire l'angle formé par ce cône en fonction du rayon du cylindre utilisé. Plus l'angle formé est grand, moins l'écoulement de la poudre est bon. A titre d'exemple, les angles de repos des excipients les plus généralement utilisés en compression directe varient entre 35 et 67°, les valeurs les plus élevées étant observées avec les celluloses et les moins élevées étant observées avec les produits granulés comme le sorbitol ou le phosphate dicalcique modifié. Le lactose atomisé présente généralement un angle de repos de l'ordre de 46° (d'après STAMM A. et MATHIS C., Labo-Pharma - Problèmes et Techniques - N°252 - Mars 1976). Quant à l'amidon, celui-ci ne s'écoule pour ainsi dire pas.

Les granules conformes à l'invention possédant les caractéristiques mentionnées ci-dessus sont susceptibles d'être obtenus selon une multitude de variantes mais tout particulièrement selon un procédé caractérisé en ce qu'il comprend une étape d'atomisation d'une suspension de lactose et d'amidon. De façon surprenante et inattendue, la Demanderesse a constaté que l'atomisation appliquée à une suspension de lactose et d'amidon natif permettait d'obtenir des granules sphériques, possédant une friabilité modérée, une comprimabilité et un écoulement performant, tout en conservant des propriétés désintégrantes. Ce but n'avait pas été atteint jusqu'alors au moyen des procédés connus de l'homme du métier, et applicables à la fois au lactose et à l'amidon. Ce dernier présente en effet un inconvénient lorsque l'on utilise des procédés thermiques en milieu aqueux, qui est le risque de cuire et de perdre ainsi son caractère granulaire et donc désintégrant.

Pour procéder à l'atomisation, on prépare une suspension d'amidon dans l'eau froide, à laquelle on ajoute du lactose monohydraté. Le mélange, ayant une température comprise d'ordinaire entre 15 et 25°C, est ensuite atomisé dans un atomiseur classique connu de l'homme du métier, et en choisissant généralement une température d'entrée voisine de 160°C et un débit tel que les températures de l'air et du produit atomisé soient en sortie voisines de 65°C. Généralement, on utilisera une suspension aqueuse d'amidon à 20% en poids. Le mélange pourra également comprendre d'autres substances autres que le lactose et l'amidon, pour autant que celles-ci ne soient pas sujettes à une dégradation thermique. En ce qui concerne la teneur en eau de la suspension contenant le lactose et l'amidon, on se placera généralement à une matière sèche de 40 à 50%. Le lactose utilisé est de préférence sous forme monohydrate. En ce qui concerne l'amidon, tout amidon granulaire peut convenir. On choisira avantageusement de l'amidon de maïs, et de préférence de l'amidon de maïs dit « extra-blanc » tel que celui commercialisé par la Demanderesse sous cette appellation. La poudre atomisée peut être utilisée directement. Elle se présente sous la forme de granules parfaitement sphériques comprenant du lactose et de l'amidon coatomisés, et peut ainsi être avantageusement utilisée comme excipient, support d'additifs, agent de texture, dans les domaines alimentaires et pharmaceutiques. Bien d'autres applications des granules conformes à l'invention sont bien entendu envisageables du fait des propriétés fonctionnelles avantageuses qu'ils présentent, notamment dans les applications vétérinaires, phytosanitaires, agricoles, ainsi que dans le domaine de la détergence.

L'invention sera mieux comprise à l'aide des exemples qui suivent et des figures 1,1' et 2,2' qui s'y rapportent, lesquels se veulent illustratifs et non limitatifs.

### Exemple 1 : Préparation de granules selon l'invention et caractérisation physique.

On prépare par coatomisation selon l'invention différentes compositions de granules consistant en lactose et en amidon à des ratios respectivement de 85/15, 75/25, 50/50 et 25/75. On utilise du lactose monohydraté et de l'amidon de maïs « extra-blanc ».

Les principales caractéristiques physiques des compositions préparées sont données dans le tableau suivant, en comparaison avec des produits de l'art antérieur.

| | **25/75** | **50/50** | **75/25** | **85/15** | **Amidon de maïs natif** | **Mélange physique d'amidon de maïs standard et de lactose monohydrate** |
|---|---|---|---|---|---|---|
| Diamètre moyen (µm) | 215 | 182 | 171 | 183 | 14 | 41 |
| Masse volumique apparente (g/l) | 0.60 | 0.60 | 0.58 | 0.59 | 0.43 | 0,44 |
| Masse volumique après tassement (g/l) | 0.65 | 0.65 | 0.69 | 0.70 | 0.74 | 0,82 |
| Temps d'écoulement (s) | 3.7 | 3.8 | 4 | 5 | infini | infini |
| Angle de repos (°) | 30 | 28 | 33 | 38 | 55 | 52 |

Le diamètre moyen est mesuré au moyen d'un granulomètre laser LS de marque COULTER, par la détermination de la répartition volumique en taille des granules.

La masse volumique apparente est mesurée selon la méthode de pharmacotechnie 2.9.15 de la Pharmacopée Européenne, 3^{ème} édition.

Le temps d'écoulement est mesuré selon la méthode de pharmacotechnie 2.9.16 de la Pharmacopée Européenne, 3^{ème} édition.

L'angle de repos est mesuré sur l'appareil POWDER TESTER commercialisé par la société HOSOKAWA, selon la méthode développée par Carr.

Les granules selon l'invention se présentent sous la forme de poudres denses, ce qui est inattendu pour des compositions atomisées. Ces poudres ont des propriétés d'écoulement remarquables.

### Figures 1,1' et 2,2'

Les compositions de granules 75/25 et 85/15 telles que préparées à l'exemple 1 sont observées en microscopie électronique à balayage, et présentent une structure sphérique caractéristique.

### Exemple 2 : détermination des profils de compression des granules selon l'invention.

On effectue sur les compositions 50/50, 75/25 et 85/15, ainsi que sur une composition de lactose atomisé de l'art antérieur un profil de compression de la manière suivante :
on prépare des comprimés sur presse alternative Frogerais de type AM, équipée de poinçons concaves de diamètre 13mm, à partir d'une poudre consistant en 99,5% de compositions de granules selon l'invention et 0,5% de stéarate de magnésium.

On obtient des comprimés d'épaisseur 6mm, sur lesquels on détermine la densité et la dureté sur duromètre ERWEKA. On détermine également le temps de désintégration des comprimés selon la méthode de la pharmacopée européenne, 3^{ème} édition. Les résultats obtenus sont consignés dans le tableau suivant :

| **COMPOSITION** | **Densité** | **Dureté moyenne (N)** | **Temps de désintégration (s)** |
|---|---|---|---|
| **50/50** | 1.215 | 55 | |
| | 1.268 | 71 | |
| | 1.312 | 117 | |
| | 1.349 | 131 | 180 |
| | 1.395 | 191 | 220 |
| | | | |
| **75/25** | 1.156 | 22 | 67 |
| | 1.235 | 55 | |
| | 1.309 | 124 | 83 |
| | 1.378 | 217 | |
| | 1.454 | 363 | 321 |
| | | | |
| **85/15** | 1.142 | 28 | 56 |
| | 1.220 | 47 | |
| | 1.292 | 96 | 83 |
| | 1.363 | 180 | |
| | 1.437 | 307 | |
| | 1.475 | 431 | 321 |
| | | | |
| **Lactose atomisé** | 1.100 | 33 | 60 |
| | 1.253 | 101 | |
| | 1.333 | 181 1 | 261 |
| | 1.403 | 313 | |
| | 1.481 | 463 | **1302** |

En traçant les courbes de dureté des comprimés en fonction de leur densité, il est possible de déterminer les duretés obtenues à des densités de 1,3 et 1,4 conformément au test B :

| | **Dureté** | | |
|---|---|---|---|
| **Densité** | **50/50** | **75/25** | **85/15** |
| 1.3 | 104 | 116 | 105 |
| 1.4 | 197 | 259 | 244 |

Ces résultats permettent d'illustrer les propriétés remarquables de comprimabilité des granules selon l'invention.

### Exemple 3 : Etude de stabilité au stockage.

Afin d'évaluer la stabilité des comprimés préparés à partir de granules selon l'invention, on prépare des comprimés avec les compositions de granules précédentes : 25/75, 50/50, 75/25, dans les mêmes conditions que l'exemple 2.

On mesure le poids et la densité de ces comprimés puis on mesure à nouveau ces paramètres après stockage des comprimés pendant 2 mois à température ambiante.

L'évolution du poids, exprimée en pourcentage, donne une indication du niveau d'hygroscopicité.

L'évolution de la dureté donne une indication de la stabilité des comprimés.

On obtient les résultats suivants :

| **COMPOSITION 25/75** | | | | | |
|---|---|---|---|---|---|
| **Données initiales** | | **Evolution après deux mois de stockage** | | | |
| **POIDS** (mg) | **DURETE** (N) | **POIDS** | **EVOLUTION** | **DURETE** | **EVOLUTION** |
| 786 | 78 | 787 | 0 | 77 | 0 |

| **COMPOSITION 50/50** | | | | | |
|---|---|---|---|---|---|
| **Données initiales** | | **Evolution après deux mois de stockage** | | | |
| **POIDS** (mg) | **DURETE** (N) | **POIDS** | **EVOLUTION** | **DURETE** | **EVOLUTION** |
| 564 | 75 | 580 | +2,8% | 83 | +11 |

| **COMPOSITION 75/25** | | | | | |
|---|---|---|---|---|---|
| **Données initiales** | | **Evolution après deux mois de stockage** | | | |
| **POIDS** (mg) | **DURETE** (N) | **POIDS** | **EVOLUTION** | **DURETE** | **EVOLUTION** |
| 729 | 81 | 734 | <1% | 72 | -11% |

### Exemple 4 : Comparaison entre la friabilité des granules selon l'invention et celles des produits de l'art antérieur.

Une granulation de lactose et d'amidon est effectuée en lit d'air fluidisé selon le mode opératoire décrit par WAN L.C. et LIM K.S., STP PHARMA SCIENCES 4 (560-570) 1988.

Le mélange 50/50 conforme à l'invention est granulé par pulvérisation d'eau pure ①, d'une solution de lactose ②, d'une solution de polyvinylpyrrolidone ③ qui est le liant le plus utilisé en pharmacie, à raison de 5g pour 100g de composition selon l'invention.

On détermine la friabilité selon le test A décrit précédemment sur ces trois compositions ainsi que sur une composition de lactose granulé disponible commercialement sous l'appellation TABLETTOSE® 80.

Les résultats sont repris dans le tableau suivant :

| **COMPOSITION** | **Friabilité (test A)%** |
|---|---|
| ① granulation à l'eau | 100 (*) |
| ② granulation lactose | 100 (*) |
| ③ granulation PVP | 53 |
| 25/75 | 26 |
| 50/50 | 24 |
| 75/25 | 48 |
| 85/15 | 52 |
| Tablettose® 80 | 56 |

| | |
|---|---|
| (*) la poudre est tellement friable que la majorité des granules sont détruits lors de l'étape de tamisage préliminaire entre 100 et 200µm. | |

Il est surprenant que l'ajout d'amidon réputé pour augmenter la friabilité des granules à base de lactose ait l'effet inverse dans les compositions selon l'invention. La friabilité est aussi faible que celle obtenue par un procédé de granulation classique mais avec ajout de 5% de liant.

### Exemple 5 : Etude de la stabilité au cisaillement des compositions selon l'invention.

Les compositions 75/25 et 85/15 préparées conformément à l'exemple 1 subissent un mélange dans un appareil de type bi-vis RUBERG type HM-50. La vitesse du mélangeur est réglée au niveau 5. Le volume de remplissage est de 30 % pour le 75/25 et 15% pour le 85/15. La taille des particules des compositions testées est mesurée par tamisage, sur tamis successifs de 32, 63, 100, 160, 250 et 315 µm, avant et après mélange pendant 2, 5, 10, 15, 20 et 25 minutes.

Les résultats sont repris dans le tableau suivant :

| **75/25** | | | | | | | |
|---|---|---|---|---|---|---|---|
| **Taille des particules** | **% de composition en fonction du temps de mélange (min)** | | | | | | |
| | 0 | 2 | 5 | 10 | 15 | 20 | 25 |
| <32µm | 2,6 | 2,9 | 3 | 3,7 | 3 | 4 | 3,3 |
| <63µm | 6,1 | 6 | 6,2 | 6,9 | 7,9 | 6,6 | 6,6 |
| <100µm | 10,4 | 10,6 | 11,6 | 10,7 | 12,3 | 11,2 | 10,4 |
| <160µm | 23 | 23,4 | 24,7 | 23,5 | 24,7 | 23,7 | 22 |
| <250µm | 65,3 | 66,4 | 66,6 | 65,7 | 67,6 | 65,5 | 64,7 |
| <315µm | 91,1 | 90,8 | 91,4 | 91,6 | 93,2 | 91 | 90,4 |
| <400µm | 99,5 | 99,2 | 99,7 | 99,8 | 99,6 | 99,9 | 99,9 |

| **85/15** | | | | | | | |
|---|---|---|---|---|---|---|---|
| **Taille des particules** | **% de composition en fonction du temps de mélange (min)** | | | | | | |
| | 0 | 2 | 5 | 10 | 15 | 20 | 25 |
| <32µm | 7 | 6,5 | 7,2 | 6,9 | 7,3 | 7,3 | 7,7 |
| <63µm | 14,2 | 13,2 | 14,5 | 14 | 13,3 | 13,9 | 13,9 |
| <100µm | 26,6 | 21,2 | 22,6 | 21,9 | 21,7 | 22,2 | 21,5 |
| <160µm | 48,5 | 42 | 43,7 | 43,4 | 42,7 | 43,9 | 43,7 |
| <250µm | 89 | 87,3 | 88,2 | 88 | 88 | 88,2 | 88,6 |
| <315µm | 99,2 | 99,1 | 99,1 | 99 | 99,1 | 99,1 | 99,1 |

Il n'y a pas de modification de la granulométrie de compositions durant le mélange. Les deux vis du mélangeur ne cassent pas la poudre, puisqu'il n'y a pas de formation de fines. Ceci confirme la faible friabilité des compositions selon l'invention, illustrée par leur bonne stabilité au cisaillement.

## Revendications

1. Granules consistant en lactose et amidon **caractérisés en ce que** le ratio lactose/amidon est compris entre 90/10 et 25/75, et de préférence entre 85/15 et 50/50, **en ce qu'**ils présentent une friabilité inférieure ou égale à 80% et de préférence à 60% selon un test A consistant à soumettre lesdits granules à une action mécanique dans un friabilimètre de marque ERWEKA TA équipé d'un tambour d'abrasion tournant à une vitesse de rotation uniforme de 25 tours/minute pendant 5 minutes dans lequel ont été introduites 5 billes d'acier identiques d'un diamètre de 17mm et d'un poids de 18,87 g et une quantité de 15 g desdits granules de granulométrie comprise entre 100 et 200 microns, la valeur de la friabilité correspondant au pourcentage de poudre non retenue sur un tamis d'une largeur de maille de 100 microns, et **en ce qu'**ils présentent une structure sphérique en microscopie électronique à balayage.

2. Granules selon la revendication 1, **caractérisés en ce qu'**ils présentent un angle de repos inférieur à 45°, et de préférence inférieur à 40°.

3. Granules selon l'une ou l'autre des revendications 1 et 2, comprenant un ratio lactose/amidon compris entre 50/50 et 90/10, **caractérisés par** une comprimabilité supérieure ou égale à 70N et de préférence à 80N pour une densité de comprimés de 1.3 g/ml et/ou supérieure ou égale à 170N et de préférence à 180N pour une densité de comprimés de 1.4 g/ml, selon un test B qui consiste à mesurer les forces traduisant la résistance à l'écrasement de comprimés préparés à partir desdits granules auxquels on ajoute au préalable 0.5% en poids de stéarate de magnésium en tant que lubrifiant, lesdits comprimée cylindriques à faces convexes (rayon de courbure de 14 mm), d'un diamètre de 13 mm, d'une épaisseur de 6 mm et de masses volumiques apparentes de 1.3 et 1.4 g/ml étant comprimés à l'aide d'une presse alternative FROGERAIS de type AM équipée de poinçons ronds à faces concaves d'un diamètre égal à 13 mm, ladite presse étant réglée de telle façon que l'enfoncement du poinçon supérieur et le volume de remplissage de la matrice fassent varier la densité des comprimés, la dureté desdits comprimés étant déterminée grâce à un duromètre ERWEKA de type TBH 30 GMD.

4. Procédé de préparation de granules consistant en lactose et en amidon, **caractérisé par le fait qu'**il comprend une étape d'atomisation d'une suspension de lactose et d'amidon, le ratio lactose/amidon étant compris entre 90/10 et 25/75, et de préférence entre 85/15 et 50/50.

## Claims

1. Granules consisting of lactose and starch, **characterized in that** the lactose/starch ratio is between 90/10 and 25/75, and preferably between 85/15 and 50/50, said granules having a friability of less than or equal to 80% and preferably of less than or equal to 60% according to a test A consisting in subjecting said granules to mechanical action in a friabilimeter of the ERWEKA TA trademark equipped with a crushing chamber revolving at a uniform rotating speed of 25 revolutions/minute for 5 minutes into which 5 identical steel beads having a diameter of 17 mm and a weight of 18.87 g and a quantity of 15 g of said granules having a particle size between 100 and 200 µm are introduced, the friability value corresponding to the percentage of powder not retained by a sieve having a mesh width of 100 microns, said granules having a spherical structure under electron microscopy.

2. Granules according to claim 1, having an angle of repose of less than 45°, and preferably of less than 40°.

3. Granules according to claim 1 or 2, having a lactose/starch ratio between 50/50 and 90/10, having a tableting capacity of greater than or equal to 70N and preferably greater than or equal to 80N for a tablet density of 1.3 g/ml, and/or greater than or equal to 170N and preferably greater than or equal to 180N, for a tablet density of 1.4 g/ml, determined according to a test B consisting in measuring the force, which is representative of the crushing resistance of the tablets prepared with said granules to which 0.5% by weight of magnesium stearate has been added beforehand as a lubricant, said tablets which are cylindrical with convex faces (radius of curvature of 14 mm), having a diameter of 13 mm, a thickness of 6 mm and apparent densities of 1.3 and 1.4 g/ml, are tableted with an AM type FROGERAIS alternating press equipped with round dies with concave faces, having a diameter equal to 13 mm, the penetration of the top die and the filling volume of the bottom die are set on the press so as to vary the density of the tablets, and the corresponding hardness of said tablets is determined using an ERWEKA durometer of the TBH 30 GMD type.

4. Process for the preparation of granules consisting of lactose and starch, **characterized in that** it comprises a step of spray-drying a suspension of lactose and starch, the lactose/starch ratio being between 90/10 and 25/75, preferably between 85/15 and 50/50.

## Patentansprüche

1. Granulat, bestehend aus Lactose und Stärke, **dadurch gekennzeichnet, dass** das Lactose/Stärke-Verhältnis zwischen 90/10 und 25/75 und vorzugsweise zwischen 85/15 und 50/50 beträgt, und **dadurch**, dass es eine Brüchigkeit von weniger oder gleich 80% und vorzugsweise 60% gemäß einem Test A hat, bei dem man das Granulat einer mechanischen Einwirkung in einem Bruchfestigkeitstester der Marke ERWEKA TA unterzieht, der mit einer Abriebtrommel ausgestattet ist, die mit einer gleichmäßigen Rotationsgeschwindigkeit von 25 Umdrehungen/Minute für 5 Minuten rotiert und in die man 5 identische Stahlkugeln mit einem Durchmesser von 17 mm und einem Gewicht von 18,87 g und eine Menge von 15 g des Granulats mit einer Granulometrie zwischen 100 und 200 Mikron einbringt, wobei der Wert für die Bruchfestigkeit dem Prozentanteil an Pulver entspricht, das auf einem Sieb mit einer Maschenweite von 100 Mikron nicht zurückgehalten wird, und **dadurch**, dass sie eine sphärische Struktur bei der Rasterelektronenmikroskopie zeigen.

2. Granulat nach Anspruch 1, **dadurch gekennzeichnet, dass** es einen Ruhewinkel von weniger als 45° und vorzugsweise weniger als 40° aufweisen.

3. Granulat nach dem einen oder anderen der Ansprüche 1 und 2, umfassend ein Lactose/Stärke-Verhältnis zwischen 50/50 und 90/10, **gekennzeichnet durch** eine Komprimierbarkeit von größer oder gleich 70 N und vorzugsweise 80 N bei einer Tablettendichte von 1,3 g/ml und/oder größer oder gleich 170 N und vorzugsweise 180 N bei einer Tablettendichte von 1,4 g/ml gemäß einem Test B, bei dem man die Kräfte misst, welche die Druckfestigkeit von Tabletten angeben, die aus dem Granulat, dem man zuvor 0,5 Gew.-% Magnesiumstearat als Gleitmittel beigefügt hat, hergestellt wurden, wobei die zylindrischen Tabletten mit konvexen Seiten (Krümmungsradius 14 mm), einem Durchmesser von 13 mm, einer Dicke von 6 mm und anscheinenden Dichte von 1,3 bis 1,4 g/ml mithilfe einer alternativen Presse FROGERAIS Typ AM gepresst wurden, die mit runden Stanzen mit konkaven Seiten und einem Durchmesser von 13 mm ausgestattet ist, wobei die Presse derart reguliert wird, dass die Eindringtiefe der oberen Stanze und das Füllvolumen der Matrize zur einer Variation in der Dichte der Tabletten führen, wobei die Härte der Tabletten mit einem ERWEKA-Durometer des Typs TBH 30 GMD bestimmt wird.

4. Verfahren zur Herstellung von Granulat, bestehend aus Lactose und Stärke, **dadurch gekennzeichnet, dass** es einen Schritt der Zerstäubung einer Suspension von Lactose und Stärke umfasst, wobei das Lactose/Stärke-Verhältnis zwischen 90/10 und 25/75 und vorzugsweise zwischen 85/15 und 50/50 liegt.
